# EUROPEAN PATENT SPECIFICATION

(11) **EP 1 706 121 B1**
(45) Date of publication and mention of the grant of the patent: **03.09.2008**
(21) Application number: 05701469.8
(22) Date of filing: 10.01.2005
(51) Int. Cl.: A61K 31/55, A61P 25/28

(54) **NEUTRAL ENDOPEPTIDASE (NEP) AND HUMAN SOLUBLE ENDOPEPTIDASE (HSEP) INHIBITORS FOR PROPHYLAXIS AND TREATMENT OF NEURODEGENERATIVE DISORDERS**
NEUTRALE ENDOPEPTIDASE (NEP) UND HUMANE LÖSLICHE ENDOPEPTIDASE (HSEP) HEMMER FÜR DIE PROPHYLAXE UND BEHANDLUNG VON NEURODEGENERATIVEN ERKRANKUNGEN
INHIBITEURS DE L'ENDOPEPTIDASE NEUTRE ET DE L'ENDOPEPTIDASE SOLUBLE HUMAINE POUR LE TRAITEMENT ET LA PREVENTION DES MALADIES NEURODÉGÉNÉRATIVES

(30) Priority: 12.01.2004 US 535538 P; 12.01.2004 EP 04100067
(43) Date of publication of application: 04.10.2006
(73) Proprietor: Solvay Pharmaceuticals B.V., 1381 CP Weesp (NL)
(72) Inventor: WESKE, Michael, IPSI Department, NL-1381 CP Weesp (NL); TURSKI, Lechoslaw A., IPSI Department, NL-1381 CP Weesp (NL); IKONOMIDOU, Hrissanthi, IPSI Department, NL-1381 CP Weesp (NL); ZIEGLER, Dieter, IPSI Department, NL-1381 CP Weesp (NL)
(74) Representative: Verhage, Marinus
(86) International application number: PCT/EP2005/050075
(87) International publication number: WO 2005/067937

(56) References cited:
- WO-A-02/094176
- WO-A-20/04082637
- US-A- 5 677 297
- US-A- 5 952 327
- US-A1- 2002 013 307
- TABRIZCHI R: "SLV-306 SLOVAY" CURRENT OPINION IN INVESTIGATIONAL DRUGS, PHARMAPRESS, US, vol. 4, no. 3, March 2003 (2003-03), pages 329-332, XP009029392 ISSN: 1472-4472

## Description

The invention relates to a novel use of known benzazepine, benzoxazepine, benzothiazepine-N-acetic acid and phosphono-substituted benzazepinone derivatives having neutral endopeptidase (NEP) and/or human soluble endopeptidase (hSEP) inhibitory activity. The compounds of the invention are useful for the preparation of pharmaceutical compositions for prophylaxis and treatment of acute neuronal degeneration.

The invention relates to the use of a compound disclosed herein for the manufacture of a medicament giving a beneficial effect. A beneficial effect is disclosed herein or apparent to a person skilled in the art from the specification and general knowledge in the art. The invention also relates to the use of a compound of the invention for the manufacture of a medicament for treating or preventing a disease or condition. More particularly, the invention relates to a new use for the treatment of a disease or condition disclosed herein or apparent to a person skilled in the art from the specification and general knowledge in the art. In embodiments of the invention specific compounds disclosed herein are used for the manufacture of a medicament.

WO 02/094176 refers to the use of certain compounds with a combined inhibitory activity on neutral endopeptidase, and a novel metalloprotease, designated IGS5: 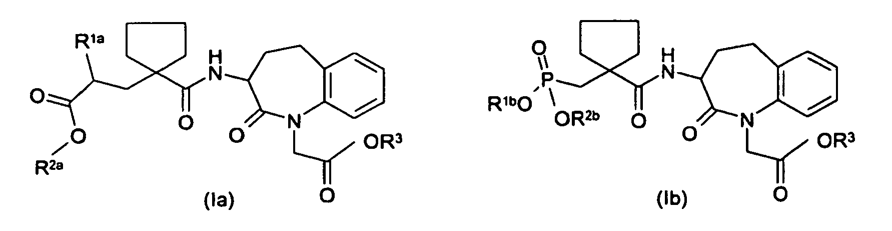 for the treatment and or prophylaxis of hypertension, including secondary forms of hypertension such as renal or pulmonary hypertension, heart failure, angina pectoris, arrhythmias, myocardial infarction, cardiac hypertrophy, cerebral ischemic, peripheral vascular disease, subarachnoidal hemorrhage, chronic obstructive pulmonary disease (COPD), asthma, renal disease, atherosclerosis, and pain in colorectal cancer or prostate cancer.

WO 2004/082637, filed on March 18, 2004, and published on September 30, 2004, discloses a method for the prophylaxis or treatment of a very large number of pathological conditions, comprising administering an aldosterone receptor antagonist and an ECE inhibitor. Among the pathological conditions listed are cerebrovascular disease susch as stroke, cerebral infarction and neuronal death, cerebral ischemia and cerebral vasospasm. Preferred ECE inhibitors comprise SLV306, its active metabolite KC-12615, and KC-90095-1-AC.

In patent application US 20030045449 it is described that matrix-metalloprotease inhibitors are useful for the treatment of neurodegenerative disorders. Problems associated with that invention are first that matrix-metalloprotease inhibitors comprise a broad group of protease inhibitors, and second that according to the said application the metalloproteases must be used in a pharmaceutical composition also containing a N-NOS inhibitor.

The goal of the present invention was to identify specific metalloprotease inhibitors which are of therapeutic value when administered as monotherapy.

Surprisingly, it now has been found that benzazepine, benzoxazepine, benzothiazepine-N-acetic acid and phosphono-substituted benzazepinone derivatives having neutral endopeptidase (NEP) and/or human soluble endopeptidase (hSEP) inhibitory activity are protective in animal traumatic brain injury model. This property makes them useful for the preparation of pharmaceutical compositions for prophylaxis and treatment of acute neuronal degeneration.

The compounds of the invention are known from the European patents EP 0 733 642 EP 0 916 679 and EP 1 468 010, containing detailed syntheses, and can be described by the general formula (1): 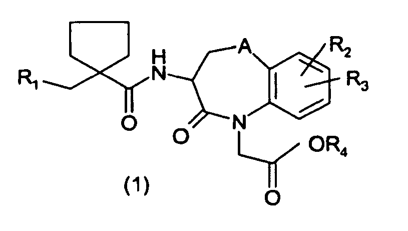 wherein:
R₁ stands for a group with formula (2) or (3): 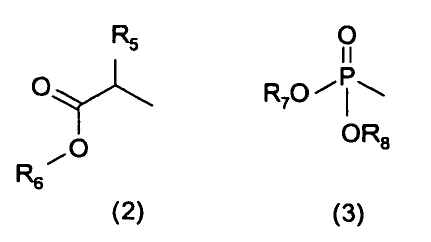
A represents CH₂, O or S,
R₂ and R₃ independently represent hydrogen or halogen,
R₄ and R₆ independently represent hydrogen or a biolabile carboxylic ester forming group;
R₅ is selected from the group consisting of (C₁-C₆)alkoxy(C₁-C₆)alkyl which may be substituted by a (C₁-C₆)alkoxy, phenyl-(C₁-C₆)-alkyl and phenyloxy-(C₁-C₆)-alkyl wherein the phenylgroup may be substituted with (C₁-C₆)alkyl, (C₁-C₆)-alkoxy or halogen, and naphtyl-(C₁-C₆)-alkyl,
R₇ and R₈ independently represent hydrogen or a group forming a biolabile phosphonic acid ester

To the invention belong all compounds having formula (1), racemates, mixtures of diastereomers and the individual stereoisomers, and also include pharmacologically acceptable salts thereof. Thus compounds in which the substituents on potentially asymmetrical carbon atoms are in either the R-configuration or the S-configuration belong to the invention.

Pharmaceutically acceptable salts may be obtained using standard procedures well known in the art, for example by mixing a compound of the present invention with a suitable metal cation or an organic base, for instance an amine.

This objective can be achieved by preparing the metal salt of the compounds with the general formula (1) as mentioned above wherein the metal ion is a lithium ion or a bivalent metal ion. Preferred bivalent metal salts are calcium, magnesium and zinc salts. Most preferred is the calcium salt.

The invention relates to the use of a compound of general formula (1), as defined above, for the preparation of pharmaceutical compositions for prophylaxis and treatment of conditions selected from the group consisting of traumatic brain injury, acute disseminated encephalo-myelitis, epilepsy related brain damage, spinal cord injury, bacterial or viral meningitis and meningo-encephalitis, prion diseases, poisonings with neurotoxic compounds, and radiation-induced brain damage, and for prophylaxis of ischemic stroke, with the proviso that said pharmaceutical compositions do not contain an aldosterone receptor antagonist.

Further embodiments of the invention are defined in the dependent claims.

The invention particularly relates to the use of compounds having general formula (4): 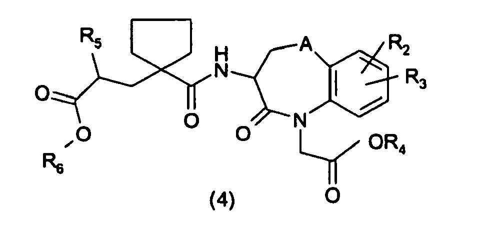 wherein the symbols have the meanings as given above.

More particular, the invention relates to the use of compounds having general formula (5): 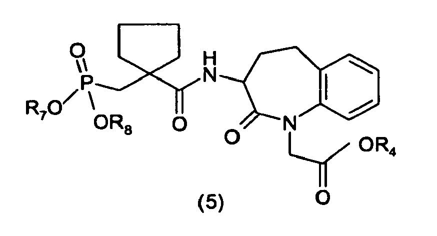 wherein the symbols have the meanings as given above.

The most preferred active substances used according to the present invention are:
- (2*R*)-2-{[1-({[(3S)-1-(carboxymethyl)-2-oxo-2,3,4,5-tetrahydro-1*H*-1-benzazepin-3-yl]amino}carbonyl)cyclopentyl]methyl}-4-phenylbutanoic acid (6): 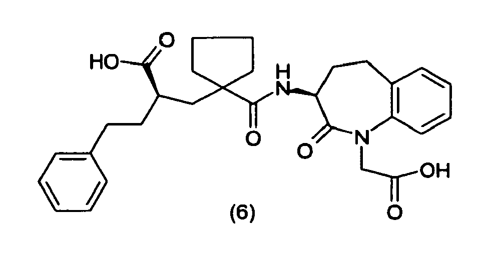
- (2*R*)-2-{[1-({[(3S)-1-(carboxymethyl)-2-oxo-2,3,4,5-tetrahydro-1*H-*1-benzazepin-3-yl]amino}carbonyl)cyclopentyl]methyl}-4-(1-naphthyl)butanoic acid (7): 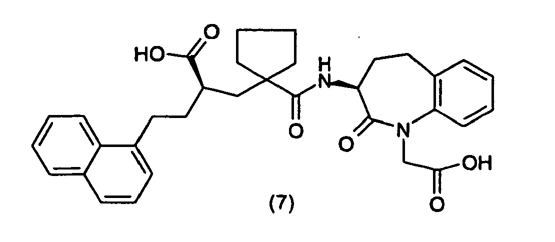
- *tert*-butyl-((3*S*)-3-{[(1-{[(benzyloxy)(ethoxy)phosphoryl]methyl)cyclopentyl) carbonyl]amino}-2-oxo-2,3,4,5-tetrahydro-1*H*-1-benzazepin-1-yl)acetate (8):

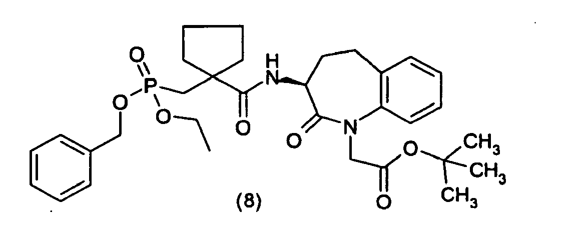

### PHARMACEUTICAL COMPOSITIONS

The compounds of the invention can be brought into forms suitable for administration by means of usual processes using auxillary substances such as liquid or solid carrier material. The pharmaceutical compositions of the invention may be administered enterally, orally, parenterally (intramuscularly or intravenously), rectally or locally (topically). They can be administered in the form of solutions, powders, tablets, capsules (including microcapsules), ointments (creams or gel) or suppositories. Suitable excipients for such formulations are the pharmaceutically customary liquid or solid fillers and extenders, solvents, emulsifiers, lubricants, flavorings, colorings and/or buffer substances. Frequently used auxiliary substances which may be mentioned are magnesium carbonate, titanium dioxide, lactose, mannitol and other sugars, talc, lactoprotein, gelatin, starch, cellulose and its derivatives, animal and vegetable oils such as fish liver oil, sunflower, groundnut or sesame oil, polyethylene glycol and solvents such as, for example, sterile water and mono- or polyhydric alcohols such as glycerol.

Compounds of the present invention are generally administered as pharmaceutical compositions. Types of pharmaceutical compositions that may be used include but are not limited to tablets, chewable tablets, capsules, solutions, parenteral solutions, suppositories, suspensions, and other types disclosed herein or apparent to a person skilled in the art from the specification and general knowledge in the art. The pharmaceutical compositions of the invention do not contain an aldosterone receptor agonist.

In embodiments of the invention, a pharmaceutical pack or kit is provided comprising one or more containers filled with one or more of the ingredients of a pharmaceutical composition of the invention. Associated with such container(s) can be various written materials such as instructions for use, or a notice in the form prescribed by a governmental agency regulating the manufacture, use or sale of pharmaceuticals products, which notice reflects approval by the agency of manufacture, use, or sale for human or veterinary administration.

Very specific formulations suitable for the compounds of the invention have been described in the patent applications WO 03/068266 and WO 04/062692.

The specific compounds described above are intended to further illustrate the invention in more detail, and therefore are not deemed to restrict the scope of the invention in any way.

### TRAUMATIC BRAIN INJURY: DELAYED NEURONAL DEATH

### Methods

**Contusing device.** The contusing device consisted of a stainless steel tube, 40 cm in length, perforated at 1 cm intervals to prevent air compression in the tube. Wistar rats, 230-270 g, were anesthetized with chloral hydrate, 400 mg/kg i.p., a craniotomy over the right hemisphere was made, the device guiding a falling weight onto the footplate resting upon the surface of the dura was placed perpendicular to the surface of the skull, and a force of 380 g x cm produced by a 20 g weight was selected to produce brain contusion. A maximum of 2.5 mm depression of the brain surface was allowed to avoid mechanical puncture of the dura. The center of the footplate was stereotaxically positioned 1.5 mm posterior and 2.5 mm lateral to the bregma. The rats underwent perfusion fixation 3 days after brain injury with a solution containing 4% paraformaldehyde in phosphate buffer.

**Intracerebroventricular (i.c.v.) injections:** Compounds were administered i.c.v. by means of a Hamilton syringe in a volume of 5-15 µl. Injections were performed over 5 min, 15 min - 8 hrs after trauma using the following stereotaxic coordinates: AP= - 0.5 mm, L= - 2 mm and V= -5.5 in relation to bregma (Swanson, L. W. (1992) Brain Maps: Structure of the Rat Brain , Elsevier, Amsterdam).

**Intravenous injections:** Compounds were administered i.v. using a 1ml syringe attached to a 26 gauge needle. The needle was inserted into the left femoral vein following a small skin incision. Compounds were administered in a volume of 1 ml/kg body weight over 30 sec.

**Morphometric analysis in hippocampus.** The damage in the hippocampal CA3 subfield was determined stereologically at 5 different rostrocaudal levels extending from 10.21 to 11.21 mm (Swanson, L. W. (1992) Brain Maps: Structure of the Rat Brain, Elsevier, Amsterdam) and throughout its mediolateral axis three days after traumatic injury. To quantitatively assess neuronal loss in the hippocampus, stereological disector technique (Cruz-Orive, L. M. & Weibel, E. R. (1990) Am. J. Physiol. 258, L148-L156) was used to estimate numerical density (Nᵥ) of pyramidal neurons. An unbiased counting frame (0.05 mm x 0.05 mm; disector height 0.01 mm) and a high-aperture objective (x40) were used for sampling. Normal neurons were identified by the presence of the typical nuclei with clear nucleoplasm and distinct nucleolus surrounded by cytoplasm containing Nissl substance. The border between CA2 and CA3 subfields was considered as the point where the looser arrangement of large pyramidal cells goes into densely packed pyramidal cells of the subfield CA3. An arbitrary line connecting the lateral ends of the dentate granule cell layers was considered a junction between subfields CA3 and CA4.

The compounds of the invention are useful in prophylaxis and treatment of neurodegenerative disorders such as e.g. traumatic brain injury, acute disseminated encephalo-myelitis, epilepsy related brain damage, spinal cord injury, bacterial or viral meningitis and meningoencephalitis, prion diseases, poisonings with neurotoxic compounds, radiation-induced brain damage, and for the prophylaxis of ischemic stroke.
The dosage expediently administered is 0.001 - 1000 mg/kg, preferably 0.1-100 mg/kg of patient's bodyweight.

### PHARMACOLOGICAL TESTRESULTS

In the traumatic brain injury model described above, the compounds with the formulae 6 and 7 elicited dose-dependent neuroprotective effects. Those effects were still evident when the compounds with the formulae 6 and 7 were administered i.c.v. up to 8 hrs after trauma.

### Dose response of the neuroprotective effect of test compounds

Dose responses of the neuroprotective effect of the compounds with the formulae 6 and 7 when administered i.c.v. 15 min after trauma to adult Wistar rats were measured. Neuronal densities were determined in the CA3 hippocampal subfield as described in the methods. Densities of CA3 neurons ± Standard Deviation (= SED) in 6 stereotactic levels in the left non-traumatized side of vehicle treated rats and the traumatized right side of vehicle treated rats and in rats treated with the compounds with the formulae 6 and 7 were measured and the results listed in table 1 below. In all of the following tables the numbers ("n") indicate the number of rats per group, where applicable.

**Table 1: Neuronal densities CA3 hippocampus, cells x 10³/mm³**

| **Stereo-tactic level** | **Vehicle left; (n=8)** | **Vehicle right;** (**n=8)** | **COMPOUND FORMULA (6) (N=8)** | **COMPOUND FORMULA (7) (N=8)** |
|---|---|---|---|---|
| **10.21** | 148.57±2.23 | 90.29±5.54 | 112.5±11.40 | 107.25±15.19 |
| **10.41** | 154.29±3.73 | 84.86±7.29 | 103.75±11.80 | 100.25±14.71 |
| **10.61** | 158.86±3.44 | 77.71±5.82 | 101.75±14.80 | 93.50±16.96 |
| **10.81** | 155.71±4.07 | 76.57±13.45 | 98.50±10.68 | 93.50±12.36 |
| **11.01** | 150.86±1.95 | 85.71±10.98 | 96.75±13.98 | 101.50±18.81 |
| **11.21** | 148.29±1.38 | 92.86±8.71 | 101.75±15.17 | 107.75±16.51 |

Injection of vehicle into the right cerebral ventricle of rats subjected to head trauma resulted in the decrease of neuronal densities in the CA3 hippocampus up to 48 % of control values, while injection of 10 µg of either the compound with the formula (6) or that with formula (7) partially prevented hippocampal neuronal loss. Analysis of variance ("ANOVA") revealed that there was a significant protective effect of treatment on neuronal loss in the CA3 hippocampus for both test substances.

### Activity after intravenous (i.v.) administration

Injection of vehicle resulted in the decrease of neuronal densities in the CA3 hippocampus up to 53 % of control values, while injection of 30 or 300 mg/kg of the test substance of formula (7) partially prevented hippocampal neuronal loss, with the dose of 300 mg/kg being most effective. Analysis of variance ("ANOVA") revealed that there was a significant protective effect of treatment on neuronal loss in the CA3 hippocampus for both tested doses of the test substance. (P<0.001; n = 8 per group). ANOVA also revealed that the dose of 300 mg/kg conferred significantly better neuroprotection than the doses of 30 mg/kg.

**Table 2: Neuronal densities CA3 hippocampus (cells x 10³/mm³), i.v. administration**

| **Stereotactic level** | **Vehicle left; (n=8)** | **Vehicle right; (n=8)** | **COMPOUND FORMULA (7) 300MG/KG (N=8)** | **COMPOUND FORMULA (7) 30 MG/KG (N=8)** |
|---|---|---|---|---|
| **10.21** | 150.50 ± 1.41 | 97.50 ± 7.39 | 126.50 ± 5.53 | 107.50 ± 6.99 |
| **10.41** | 154.25 ± 1.67 | 90.25 ± 5.60 | 117.75 ± 4.95 | 102.25 ± 7.89 |
| **10.61** | 157.25 ± 2.38 | 84.75 ± 7.09 | 110.00 ± 7.01 | 100.75 ± 9.91 |
| **10.81** | 154.00 ± 1.85 | 88.00 ± 7.17 | 106.75 ± 7.48 | 101.75 ± 9.65 |
| **11.01** | 149.00 ± 1.07 | 94.00 ± 5.45 | 116.50 ± 9.06 | 111.25 ± 7.55 |
| **11.21** | 146.00 ± 1.51 | 99.75 ± 8.24 | 125.25 ± 5.01 | 117.75 ± 10.11 |

## Claims

1. Use of a compound of the general formula (1) 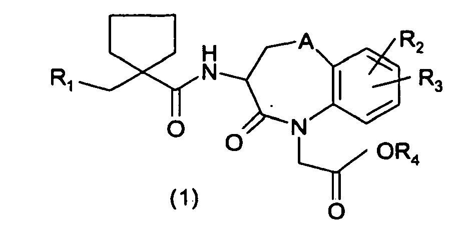 wherein:
R₁ stands for a group with formula (2) or (3): 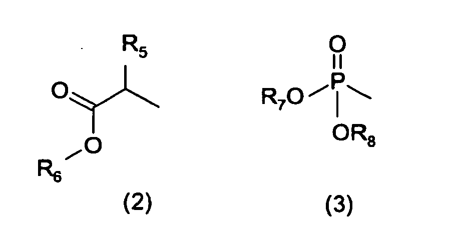
A represents CH₂, O or S,
R₂ and R₃ independently represent hydrogen or halogen,
R₄ and R₆ independently represent hydrogen or a biolabile carboxylic ester forming group;
R₅ is selected from the group consisting of (C₁-C₆)alkoxy(C₁-C₆)alkyl which may be substituted by a (C₁-C₆)alkoxy, phenyl-(C₁-C₆)-alkyl and phenyloxy-(C₁-C₆)-alkyl wherein the phenylgroup may be substituted with (C₁-C₆)alkyl, (C₁-C₆)-alkoxy or halogen, and naphtyl-(C₁-C₆)-alkyl,
R₇ and R₈ independently represent hydrogen or a group forming a biolabile phosphonic acid ester,
all stereoisomers, as well as pharmacologically acceptable salts thereof, for the preparation of pharmaceutical compositions for prophylaxis and treatment of conditions selected from the group consisting of traumatic brain injury, acute disseminated encephalo-myelitis, epilepsy related brain damage, spinal cord injury, bacterial or viral meningitis and meningo-encephalitis, prion diseases, poisonings with neurotoxic compounds, and radiation-induced brain damage, and for prophylaxis of ischemic stroke, with the proviso that said pharmaceutical compositions do not contain an aldosterone receptor antagonist.

2. Use as claimed in claim 1, **characterized in that** said pharmaceutical compositions contain at least one compound of the general formula (4) 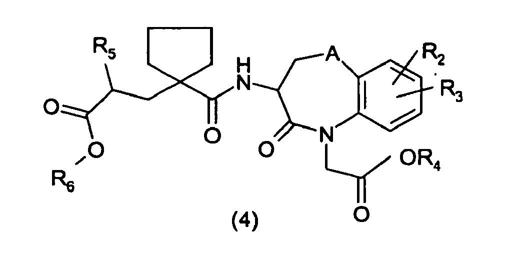 wherein the symbols have the meanings as given in claim 1, all stereoisomers, as well as pharmacologically acceptable salts thereof.

3. Use as claimed in claim 1, **characterized in that** said pharmaceutical compositions contain at least one compound of the general formula (5) 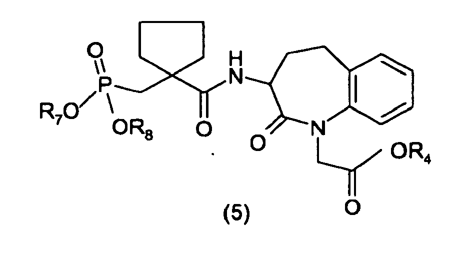 wherein the symbols have the meanings as given in claim 1, all stereoisomers, as well as pharmacologically acceptable salts thereof.

4. Use as claimed in claim 1, **characterized in that** said compound is (2*R*)-2-{[1-({[(3*S*)-1-(carboxymethyl)-2-oxo-2,3,4,5-tetrahydro-1*H-*1-benzazepin-3-yl] amino}carbonyl)cyclopentyl]methyl}-4-phenylbutanoic acid having formula (6): 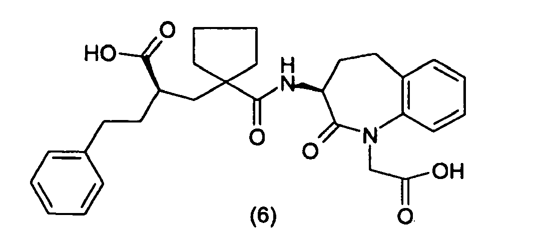 as well as pharmacologically acceptable salts thereof.

5. Use as claimed in claim 1, **characterized in that** said compound is (2*R*)-2-{(1-({[(3*S*)-1-(carboxymethyl)-2-oxo-2,3,4,5-tetrahydro-1*H-*1-benzazepin-3-yl]amino}carbonyl)cyclopentyl]methyl}-4-(1-naphthyl)butanoic acid, having formula (7): 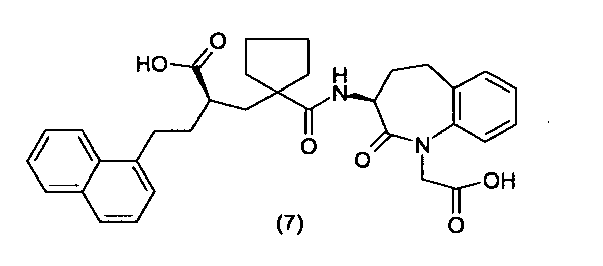 as well as pharmacologically acceptable salts thereof.

6. Use as claimed in claim 1, **characterized in that** said compound is tert-butyl-((3*S*)-3-{[(1-{[(benzyloxy)(ethoxy)phosphoryl]methyl}cyclopentyl) carbonyl]amino)-2-oxo-2,3,4,5-tetrahydro-1*H-*1-benzazepin-1-yl)acetate, having formula (8): 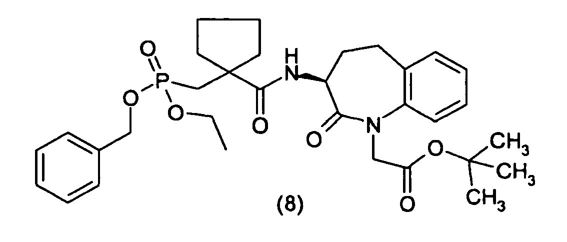 as well as pharmacologically acceptable salts thereof.

7. Use as claimed in any of the claims 1-6, **characterized in that** the pharmacologically acceptable salt is selected from the group consisting of the lithium salt, the calcium salt, the magnesium salt and the zinc salt, and that the pharmacologically acceptable salt preferably is the calcium salt.

8. Use as claimed in any of the claims 1-6, **characterized in that** said treatment is for traumatic brain injury or spinal cord injury.

9. Use as claimed in any of the claims 1 - 6, **characterized in that** said treatment is for acute disseminated encephalo-myelitis, epilepsy related brain damage, bacterial or viral meningitis and meningoencephalitis, prion diseases, poisonings with neurotoxic compounds, and radiation-induced brain damage.

10. Use as claimed in any of the claims 1 - 6, **characterized in that** said prophylaxis is for ischemic stroke.

## Patentansprüche

1. Verwendung einher Verbindung der allgemeinen Formel (1) 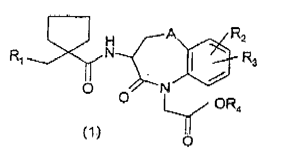 worin,
R₁ für eine Gruppe der Formel (2) oder (3) 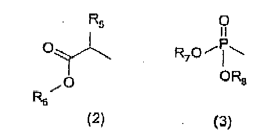 steht,
A für CH₂, O oder S steht,
R₂ und R₃ unabhängig Wasserstoff oder Halogen darstellen,
R₄ und R₆ unabhängig Wasserstoff ober eine biologisch instabile Carbonsäureester-bildende Gruppe darstellen;
R₅ ausgewählt wird aus der Gruppe bestehend aus (C₁-C₆) -Alkoxy(C₁-C₆)alkyl, welches substituiert sein kann durch ein (C₁-C₆)-Alkoxy, Phenyl-(C₁-C₆)-alkyl und Phenyloxy-(C₁-C₆)-alkyl, worin die Phenylgruppe substituiert sein kann mit (C₁-C₆) -Alkyl, (C₁-C₆) -Alkoxy oder Halogen, und Naphthyl- (C₁-C₆) -alkyl,
R₇ und R₈ unabhängig Wasserstoff oder eine Gruppe darstellen, welche einen biologisch instabilen Phosphonsäureester bildet,
aller Stereoisomere, als auch pharmakologisch annehmbarer Salze davon, für die Herstellung von pharmazeutischen Zusammensetzungen für Prophylaxe und Behandlung von Zuständen, ausgewählt aus der Gruppe bestehend aus traumatischer Hirnverletzung, akuter disseminierter Enzephalomyelitis, epilepsiebezogener Hirnschädigung, Rückenmarksverletzung, bakterieller oder viraler Meningitis und Meningoenzephalitis, Prionenkrankheiten, Vergiftungen mit neurotoxischen Verbindungen und Strahlungs-induzierter Hirnschädigung, und für Prophylaxe von ischämischem Schlaganfall, mit der Maßgabe, dass besagte pharmazeutische Zusammensetzungen keinen Aldosteron-Rezeptor-Antagonisten enthalten.

2. Verwendung wie in Anspruch 1. beansprucht, **dadurch gekennzeichnet, dass** besagte pharmazeutische Zusammensetzungen mindestens eine Verbindung der allgemeine Formel (4) enthalten 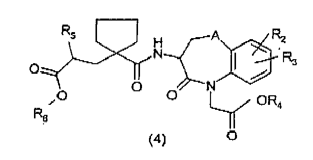 worin die Symbole die Bedeutungen wie in Anspruch 1 gegeben haben, alle Stereoisomere, als auch pharmakologisch annehmbare Salze davon.

3. Verwendung wie in, Anspruch 1 beansprucht, **dadurch gekennzeichnet, dass** besagte pharmazeutische Zusammensetzungen mindestens eine Verbindung der allgemeinen Formel (5) enthalten 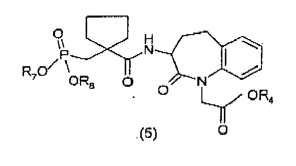 worin die Symbole die Bedeutungen wie in Anspruch 1 gegeben haben, alle Stereoisomere, als auch pharmakologisch annehmbare Salze davon.

4. Verwendung wie in Anspruch 1 beansprucht, **dadurch gekennzeichnet, dass** besagte Verbindung (2*R*)-2-([1-({[(3*S*)-1-(Carboxymethyl)-2-oxo-2,3,4,5-tetrahydro-1*H*-1-benzazepin-3-yl]-amino)carbonyl)cyclopentyl]methyl}-4-phenylbutansäure mit der Formel. (6): 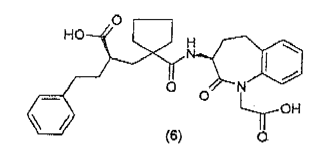 ist, als auch pharmakologisch annehmbare Salze davon.

5. Verwendung wie in Anspruch 1 beansprucht, **dadurch gekennzeichnet, dass** besagte Verbindung (2*R*)-2-{[1-({[(3*S*)-1-(carboxymethyl)-2-oxo-2,3,4,5-tetrahydro-1*H*-1-benzazepin-3-yl-amino}carbonyl)cyclopentyl]methyl}-4-(1-naphthyl)butansäure mit der Formel (7): 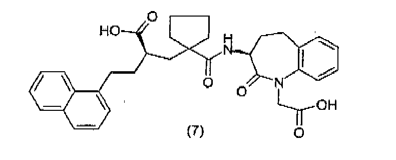 ist, als auch, pharmakologisch annehmbare Salze davon.

6. Verwendung wie in Anspruch 1 beansprucht, **dadurch gekennzeichnet**, das besagte Verbindung *tert*-Butyl-((3*S*)-3-{[(1-{[(Benzyloxy) (ethoxy) phosphoryl]methyl}cyclopentyl)-carbonyl]amino}-2-oxo-2,3,4,5-tetrahydro-1*H*-1-benzazepin-1-yl)acetat mit der Formel (8): 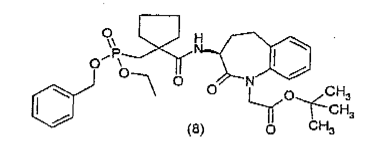 ist, als auch pharmakologisch annehmbare Salze davon.

7. Verwendung wie in einem der Ansprüche 1-6 beansprucht, **dadurch gekennzeichnet, dass** das pharmakologisch annehmbar Salz ausgewählt wird aus der Gruppe bestehend aus dem Lithiumsalz, dem Calciumsalz, dem Magnesiumsalz und dem Zinksalz, und dass das pharmakologisch annehmbare Salz vorzugsweise das Calciumsalz ist.

8. Verwendung wie in einem der Ansprüche 1-6 beansprucht, **dadurch** gekennzeichet, dass besagte Behandlung für traumatische Hirnverletzung oder Rückenmacksverletzung ist.

9. Verwendung wie in einem der Ansprüche 1-6 beansprucht, **dadurch gekennzeichnet, dass** besagte Behandlung für akute disseminierte Enzephalomyelitis, epilepsiebezogene Hirnschädigung, bakterielle oder virale Meningitis und Meningoenzcphalitis, Prionenkrankheiten, Vergiftungen mit neurotoxischen Verbindungen und. Strahlungs-induzierte Hirnschädigung ist.

10. Verwendung wie in einem der Ansprüche 1-6 beansprucht, **dadurch gekennzeichnet, dass** besagte Prophylaxe für ischämischen Schlaganfall ist.

## Revendications

1. Utilisation d'un composé de formule générale (1) : 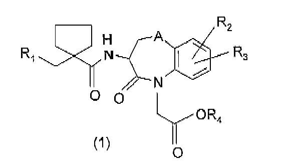 où R₁, représente un groupe de formule (2) ou (3) 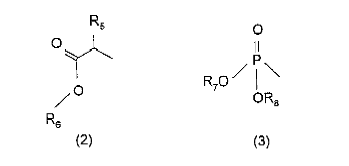 A représente CH₂, O ou S,
R₂ et R₃ représentent indépendamment l'hydrogène ou un halogène,
R₄ et R₆ représentent indépendamment l'hydrogène ou un groupe formant un ester carboxylique biolabile ;
R₅ est choisi dans le groupe consistant en (C₁-C₆)alcoxy(C₁-C₆)alkyle qui peut être substitué par un (C₁-C₆)alcoxy, phényl(C₁-C₆)alkyle et phényloxy-(C₁-C₆)alkyle, où le groupe phényle peut être substitué avec (C₁-C₆)alkyle, (C₁-C₆)alcoxy ou un halogène, et naphtyl-(C₁-C₆)alkyle,
R₇ et R₈ représentent indépendamment l'hydrogène ou un groupe formant un ester d'acide phosphonique biolabile,
de tous ses stéréoisomères, ainsi que de ses sels pharmacologiquement acceptables, pour la préparation de compositions pharmaceutiques pour la prophylaxie et le traitement d'affections choisies dans le groupe consistant en les lésions cérébrales traumatiques, l'encéphalo-myelite aiguë disséminée, les lésions cérébrales liées à l'épilepsie, les lésions de la moelle épinière, la méningite bactérienne ou virale et la méningo-encéphalite, les maladies à prions, les empoisonnements avec des composés neurotoxiques et les lésions cérébrales induites par des rayonnements, et pour la prophylaxie du choc ischémique, à condition que lesdites compositions pharmaceutiques ne contiennent pas d'antagoniste de récepteurs d'aldostérone,

2. Utilisation selon la revendication 1, **caractérisée en ce que** lesdites compositions pharmaceutiques contiennent au moins un composé de formule générale (4) 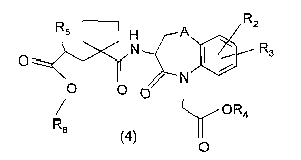 où les symboles ont les significations données dans la revendication 1, tous ses stéréoisomères, ainsi que ses sels pharmacologiquement acceptables.

3. Utilisation selon la revendication 1, **caractérisée en ce que** lesdites compositions pharmaceutiques contiennent au moins un composé de formule générale (5) 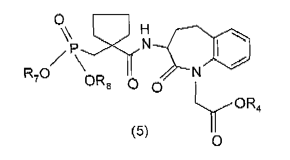 où les symboles ont les significations données dans la revendication 1, tous ses stéréoisomères ainsi que ses sels pharmacologiquement acceptables.

4. Utilisation selon la revendication 1, **caractérisée en ce que** ledit composé est l'acide (*2R*)*-*2*-*{[1-({[(3S)-1-(carboxyméthyl)-2-oxo-2,3,4,5-tétrahydro-1*H*-benzazépin-3-yl]amino}carbonyl)cyclopentyl]-méthyl}-4-phénylbutanoïque ayant la formule (6) : 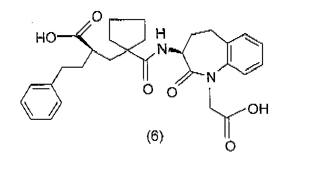 ainsi que ses sels pharmacologiquement acceptables.

5. Utilisation selon la revendication 1, **caractérisée en ce que** ledit composé est l'acide (*2R*)-2-{[1-({[(3S)-1-(carboxyméthyl)-2-oxo-2,3,4,5-tétrahydro-1*H-*1-benzazépin-3-yl]amino}carbonyl)cyclopentyl]-méthyl}-4-(1-naphtyl)butanoïque ayant la formule (7) : 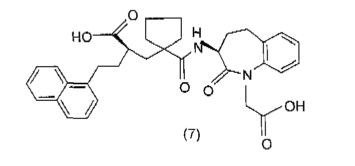 ainsi que ses sels pharmacologiquement acceptables.

6. Utilisation selon la revendication 1, **caractérisée en ce que** ledit composé est le ((3S)-3-{[(1-{[(benzyloxy)(éthoxy)phosphoryl]-méthyl}cyclopentyl)carbonyl]amino}-2-oxo-2,3,4,5-tétrahydro-1*H*-1 benzazépin-1-yl)acétate de *tert-*butyle, ayant la formule (8) : 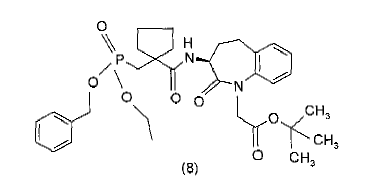 ainsi que ses sels pharmacologiquement acceptables.

7. Utilisation selon l'une quelconque des revendications 1-6, **caractérisée en ce que** le sel pharmacologiquement acceptable est choisi dans le groupe consistant en le sel de lithium, le sel de calcium, le sel de magnésium et le sel de zinc, et **en ce que** le sel pharmacologiquement acceptable est de préférence le sel de calcium.

8. Utilisation selon l'une quelconque des revendications 1-6, **caractérisée en ce que** ledit traitement est destiné aux lésions cérébrales traumatiques ou aux lésions de la moelle épinière.

9. Utilisation selon l'une quelconque des revendications 1-6, **caractérisée en ce que** ledit traitement est destiné à l'encéphalomyélite aigüe disséminée, aux lésions cérébrales liées à l'épilepsie, à la méningite bactérienne ou virale et à la méningo-encéphalite, aux maladies à prions, aux empoisonnements avec des composés neurotoxiques et aux lésions cérébrales induites par des rayonnements.

10. Utilisation selon l'une quelconque des revendications 1-6, **caractérisée en ce que** ladite prophylaxie est destinée au choc ischémique.
